# EUROPÄISCHE PATENTSCHRIFT

(11) **EP 0 935 669 B1**
(45) Veröffentlichungstag und Bekanntmachung des Hinweises auf die Patenterteilung: **31.07.2002**
(21) Anmeldenummer: 97923056.2
(22) Anmeldetag: 12.05.1997
(51) Int. Cl.: C12P 41/00, C12P 13/02, C07C 259/06, C07C 209/56, C07C 211/02, C07C 211/16, C07C 211/27

(54) **ENZYMATISCHE SYNTHESE OPTISCH AKTIVER HYDROXAMSÄUREN UND IHRE UMSETZUNG ZU OPTISCH AKTIVEN PRIMÄREN AMINEN DURCH LOSSEN-UMLAGERUNG**
ENZYMATIC SYNTHESIS OF OPTICALLY ACTIVE HYDROXAMIC ACIDS AND THEIR CONVERSION INTO OPTICALLY ACTIVE PRIMARY AMINES BY LOSSEN TRANSPOSITION
SYNTHESE ENZYMATIQUE D'ACIDES HYDROXAMIQUES OPTIQUEMENT ACTIFS ET LEUR CONVERSION EN AMINES PRIMAIRES OPTIQUEMENT ACTIVES PAR TRANSPOSITION DE LOSSEN

(30) Priorität: 20.05.1996 DE 19620189; 26.08.1996 DE 19634446
(43) Veröffentlichungstag der Anmeldung: 18.08.1999
(73) Patentinhaber: FRAUNHOFER-GESELLSCHAFT ZUR FÖRDERUNG DER ANGEWANDTEN FORSCHUNG E.V., 80636 München (DE)
(72) Erfinder: HIRRLINGER, Beate, D-73730 Esslingen (DE); STOLZ, Andreas, D-71069 Sindelfingen (DE); KNACKMUSS, Hans-Joachim, D-71229 Leonberg (DE)
(86) Internationale Anmeldenummer: EP9702413
(87) Internationale Veröffentlichungsnummer: WO9744480

(56) Entgegenhaltungen:
- WO-A-95/02574
- DATABASE WPI Section Ch, Week 8943 Derwent Publications Ltd., London, GB; Class D16, AN 89-312215 XP002040057 & JP 01 228 468 A (KAO CORP) , 12.September 1989
- CHEMICAL ABSTRACTS, vol. 124, no. 21, 20.Mai 1996 Columbus, Ohio, US; abstract no. 282729, HIRRLINGER, BEATE ET AL: "Purification and characterization of an enantio-selective amidase from Rhodococcus erythropolis MP50." XP002040055 & BIOCHEM. ENG. 3, INT. SYMP., 3RD (1995), 43-5. EDITOR(S): SCHMID, ROLF D. PUBLISHER: UNIVERSITAET STUTTGART, INSTITUT FUER TECHNISCHE BIOCHEMIE, STUTTGART, GERMANY. CODEN: 62OTAD, 1995,
- JOURNAL OF THE CHEMICAL SOCIETY, CHEMICAL COMMUNICATIONS, 1946, LETCHWORTH GB, Seiten 25-27, XP002040053 A. CAMPBELL ET AL.: "Retention of asymmetry during the Beckmann,Lossen and Curtius changes."
- J. AMER. CHEM. SOC. (1968), 90(6), 1638-43 CODEN: JACSAT, XP002040054 HOARE, D. G. ET AL: "The reaction of hydroxamic acids with water-soluble carbodiimides. A lossen rearrangement"
- CHEMICAL ABSTRACTS, vol. 112, no. 13, 26.März 1990 Columbus, Ohio, US; abstract no. 118447, UCHINO, NOBUHIKO ET AL: "Preparation of o-aminophenols by one-pot reaction of salicylhydroxamic acids" XP002040056 & JP 01 242 560 A (FUJI PHOTO FILM CO., LTD., JAPAN)

## Beschreibung

Die Erfindung betrifft die enzymatische Synthese optisch aktiver Hydroxamsäuren und ihre Umsetzung zu optisch aktiven primären Aminen durch Lossen-Umlagerung.

Hydroxamsäuren stellen pharmazeutisch hochinteressante Verbindungen dar. Es existieren Hydroxamsäurederivate mit antibakteriellen und fungiziden Eigenschaften (Duda et al., 1965, Hase et al., 1971) Alkylaminopropionhydroxamsäuren zeigen hypotensive Eigenschaften (Coutts et al., 1971). Desweiteren wurden für Hydroxamsäuren hypocholesterinämische Wirkungen nachgewiesen (Ludwig et al., 1967). p-Butoxyphenylacethydroxamsäure besitzt endzündungshemmende Wirkung (Dell et al., 1971) und wird in der Humanmedizin eingesetzt. Einige Hydroxamsäuren wurden auf ihre Wirksamkeit gegen Malaria untersucht (Hynes, 1970; Hynes & Hack, 1972).

Von besonderer Bedeutung sind jedoch enantiomerenreine Hydroxamsäuren. Gegenüber den Racematen weisen sie eine höhere pharmakologische Aktivität auf. Desweiteren erschließen sie durch die Lossen-Umlagerung auch einen neuen Weg zu chiralen primären Aminen. Auch diese Substanzklasse ist pharmakologisch von großer Bedeutung. So werden z.B. chirale β-Aminoalkohole in großen Mengen als β-Adrenozeptor-Antagonisten, kurz β-Blocker, eingesetzt.

Die Herstellung von enantiomerenreinen Hydroxamsäuren durch klassische Verfahren der organischen Chemie ist jedoch aufwendig. Sie erfordert in der Regel eine Racemattrennung oder den Einsatz von metallorganischen Katalysatoren, die jedoch zur Herstellung von Arzneimitteln im allgemeinen ungeeignet sind. Solche Verfahren sind beispielsweise in der DE-PS 2 400 531, der EP-A-203 379 sowie der EP-A-268 215 beschrieben.

Somit liegt der Erfindung die Aufgabe zugrunde, ein neues Verfahren zur Herstellung optisch aktiver Hydroxamsäuren bereitzustellen, bei dem die vorgenannten Probleme nicht auftreten.

Überraschenderweise wurde nun gefunden, daß Acyltransferasen Amide, Carbonsäureester und Carbonsäuren, die an den α-Kohlenstoffatomen ein Chiralitätszentrum besitzen, in Gegenwart von Hydroxylamin enantioselektiv zu optisch aktiven Hydroxamsäuren umsetzen können.

Gegenstand der Erfindung ist daher ein Verfahren zur Herstellung optisch aktiver Hydroxamsäuren der allgemeinen Formel 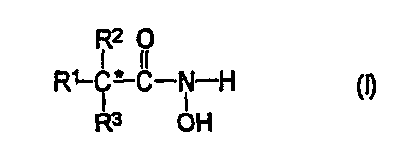 worin R¹, R² und R³ verschieden sind und für einen cyclischen oder linearen, aliphatischen oder aromatischen, substituierten oder unsubstituierten Kohlenwasserstoffrest, der ggf. Heteroatome enthalten kann, stehen, das dadurch gekennzeichnet ist, daß man ein Racemat aus chiralen Amiden, Carbonsäureestern oder Carbonsäuren der allgemeinen Formel: 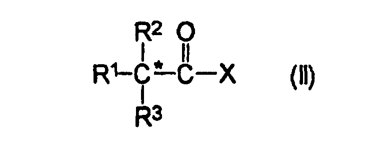 worin R¹, R² und R³ wie oben definiert sind und X für -NH₂, -OR oder -OH steht, wobei R ein beliebiger organischer Rest ist,
mit Hydroxylamin NH₂OH in Gegenwart einer Acyltransferase umsetzt und anschließend die gebildete optisch aktive Hydroxamsäure (I) von dem nichtumgesetzten Enantiomeren der allgemeinen Formel (II) abtrennt.

Geeignete Acyltransferasen, d.h. Enzyme, die den Acylrest auf das Hydroxylamin übertragen, sind an sich bekannt. Sie können beispielsweise aus den folgenden Mikroorganismen isoliert werden: Mycobacteriaceae, Mycobacterium smegmatis, Pseudomonas aeruginosa, Arthrobacter, Aspergillus nidulans, Bradyrhizobium japonicum, Brevibacterium sp. R312 Methylophilus methylotrophus, Pseudomonas chlororaphis, Pseudomonas fluorescens, Rhodococcus rhodochrous J1 und Rhodococcus erythropolis MP50.

Als ganz besonders geeignet erweist sich die aus Rhodococcus erythropolis MP50 isolierte Acyltransferase. Von allen untersuchten Amidasen hatte diese die höchsten spezifischen Aktivitäten und das breiteste Substratspektrum. Amidase- und Acyltransferaseaktivität sind im selben Enzym zu finden. Auch die Amidase-Aktivität ist hoch, die Acyltransferase-Aktivität ist jedoch deutlich höher, vermutlich da Hydroxylamin der bessere Akzeptor als Wasser für den Acylrest ist. Somit kann durch Verwendung dieser speziellen Acyltransferase , d.h. der aus Rhodococcus erythropolis MP50 isolierten Acyltransferase, die Hydrolyse der als Ausgangsmaterial eingesetzten Amide und Ester weitgehend vermieden werden.

Bei der Durchführung des erfindungsgemäßen Verfahrens kann sowohl ein Rohextrakt der Acyltransferase aus den genannten Mikroorganismen als auch eine gereinigte Form eingesetzt werden. Die Verwendung des Rohextrakts weist den Vorteil auf, daß die Stufe der Reinigung des Enzyms unterbleiben kann. Für spezielle Anwendungen empfiehlt sich jedoch die Verwendung von gereinigter Acyltransferase. Insbesondere zeigt sich, daß die katalytische Aktivität der gereinigten Acyltransferase höher ist als die des Rohextrakts, so daß reaktionsträge Ausgangsmaterialien vorzugsweise mit der gereinigten Acyltransferase umgesetzt werden.

Die Gewinnung und Reinigung der Acyltransferase aus Rhodococcus erythropolis MP50 ist in "Purification and properties of an amidase from Rhodococcus erythropolis MP50 which enantioselectively hydrolyses 2-arylpropionamides" B. Hirrlinger, A. Stolz & H.-J. Knackmuss, Journal of Bacteriology, 1996, Vol. 178(12), S. 3501-3507, beschrieben.

Die Zellen oder das gereinigte Enzym können direkt oder in immobilisierter Form eingesetzt werden. Die Immobilisation ermöglicht eine mehrfache Verwendung des Biokatalysators und vereinfacht die Aufarbeitung des Reaktionsgemisches. Im Rahmen der vorliegenden Erfindung erfolgt die Immobilisierung von ganzen Zellen oder von gereinigtem Enzym nach an sich bekannten Methoden. Ganze Zellen werden z.B. mit Alginat, κ-Carrageenan, Polyurethan oder Polyacrylamiden immobilisiert (I. Chibata, T. Tosa & T. Sato, 1983, Immobilized Cells in Preparation of Fine Chemicals, Advances In Biotechnological Processes (A.R. Liss, Hrsg.),Band 1, S. 203-222.

Gereinigtes Enzym wird entweder durch Adsorption an geeignete Trägermaterialien (Celite, Cellulose) durch ionische Bindung an ein Ionentauscherharz (DEAE-Cellulose, Sephadex), durch kovalente Bindungen an einen Carrier (poröse Glaskügelchen, Cellulose, Dextran, Agarose) oder durch Einlagern in ein Gel (Alginat, Agar, Polyacrylämid) immobilisiert (O.R. Zaborsky, 1973, Immobilized Enzymes, CRC Press, Cleveland, Ohio; M.D. Trevan, 1980, Immobilized Enzymes: Introduction and Applications in Biotechnology, Wiley, New York; W. Hartmeier, 1986, Immobilisierte Biokatalysatoren, Springer, Berlin).

Da es sich bei dem erfindungsgemäßen Verfahren um eine enzymkatalysierte Reaktion handelt, ist der pH-Wert des Reaktionsmediums zu beachten. Er sollte im allgemeinen in einem Bereich von pH = 5,5 bis 9 und vorzugsweise in einem Bereich von pH = 6,5 bis 7,5 liegen. Hierzu können geeignete Puffersysteme, wie Natriumphosphatpuffer und Tris/HCl-Puffer verwendet werden.

Prinzipiell können beliebige Amide, Carbonsäureester und Carbonsäuren als Ausgangsmaterial zur Herstellung der gewünschten Hydroxamsäuren eingesetzt werden, solange das dem Carbonylkohlenstoff benachbarte Kohlenstoffatom, d.h. das α-Kohlenstoffatom chiral ist. Das bedeutet, daß die an diesem Kohlenstoffatom vorhandenen Substituenten verschieden sein müssen. Diese Substituenten können beliebige cyclische oder lineare aliphatische oder aromatische substituierte oder unsubstituierte Kohlenwasserstoffreste sein und können ggf. Heteroatome, wie Sauerstoff, Schwefel, Stickstoff, Phosphor usw., enthalten.

Als besonders geeignet erweisen sich Substituenten, wie Methyl, Ethyl, n-Pentyl, Isopropyl, Cyclohexyl, Phenyl und Naphthyl.

Besonders geeignete Ausgangsmaterialien sind daher Alkylaminopropionsäure, p-Butoxyphenylessigsäure, 2-Phenylpropionsäure, 2-(6-Methoxy-2-naphthyl)propionsäure (Naproxen), 2-(3'-Benzoylphenyl)propionsäure (Ketoprofen), 2-Methylbutansäure und 2-(1-Naphthyl)propionsäure und deren Ester oder Amide.

Im allgemeinen ist bevorzugt, die Amide der unter die allgemeine Formel (I) fallenden Verbindungen einzusetzen, da sie reaktiver als die entsprechenden Carbonsäuren bzw. Carbonsäureester sind.

Die Konzentration der Reaktanten in dem Reaktionsmedium ist nicht besonders kritisch. Im allgemeinen setzt man die Verbindungen der Formel (I) in einer Konzentration von 0,5 bis 0,001 mol/l und vorzugsweise in einer Konzentration von 0,1 mol/l ein. Die Konzentration des Hydroxylamins beträgt im allgemeinen 1,0 bis 0,1 mol/l und vorzugsweise 0,5 bis 0,2 mol/l. Somit beträgt das Verhältnis der Verbindungen der Formel (I) zu Hydroxylamin im allgemeinen 1:10 bis 1:2 und vorzugsweise 1:5. Die Aktivität der Acyltransferase beträgt im allgemeinen 3,5 bis 37 Units/mg Protein und vorzugsweise 13 Units/mg Protein (mit 2-Phenylpropionamid als Substrat).

Der wesentliche Vorteil des erfindungsgemäßen Verfahrens ist darin zu sehen, daß die Acyltransferase selektiv lediglich die Umsetzung eines Enantiomeren des als Ausgangsmaterial eingesetzten Racemats aus optisch aktiven Amiden, Carbonsäureestern oder Carbonsäuren mit Hydroxylamin zur entsprechenden Hydroxamsäure katalysiert, während sein Spiegelbild bei der Reaktion nicht verändert wird. Somit erhält man zunächst direkt nach der Umsetzung ein Reaktionsgemisch, das die optisch reine Hydroxamsäure neben den nichtumgesetzten Enantiomeren des Ausgangsmaterials enthält. Diese beiden Komponenten können in einfacher Weise durch Ausschütteln des Reaktionsgemischs mit Essigsäureethylester, Diethylether oder Methylenchlorid voneinander getrennt werden. Dabei geht man im allgemeinen wie folgt vor:

Die wäßrige Reaktionslösung wird mit Natronlauge auf pH = 10 eingestellt. Man extrahiert das Amid mit einem der voranstehend genannten organischen Lösungsmitteln. Anschließend wird die wäßrige Phase mit Salzsäure auf pH = 2 eingestellt. Jetzt wird die Hydroxamsäure durch Ausschütteln in die organische Phase überführt und so isoliert.

Die auf diese Weise gewonnene optisch aktive Hydroxamsäure kann dann in an sich bekannter Weise durch Lossen-Umlagerung in das entsprechende optisch aktive und enantiomerenreine primäre Amin umgewandelt werden. Ein weiterer Gegenstand der Erfindung ist daher ein Verfahren zur Herstellung von optisch aktiven, primären Aminen der allgemeinen Formel 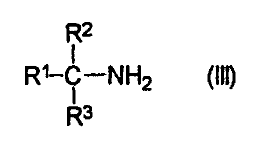 worin R¹, R² und R³ wie voranstehend definiert sind, bei dem man die, wie voranstehend beschrieben, erhaltene optisch aktive Hydroxamsäure der allgemeinen Formel (I) O-acyliert und die O-acylierte Hydroxamsäure durch Erhitzen oder durch die Behandlung mit Basen in aprotischen Lösungsmitteln zum entsprechenden Isocyanat umsetzt und das Isocyanat mit Wasser unter Abspaltung von CO₂ zum Amin der allgemeinen Formel (III) reagieren läßt.

Die Lossen-Umlagerung verläuft somit nach dem folgenden Reaktionsschema:

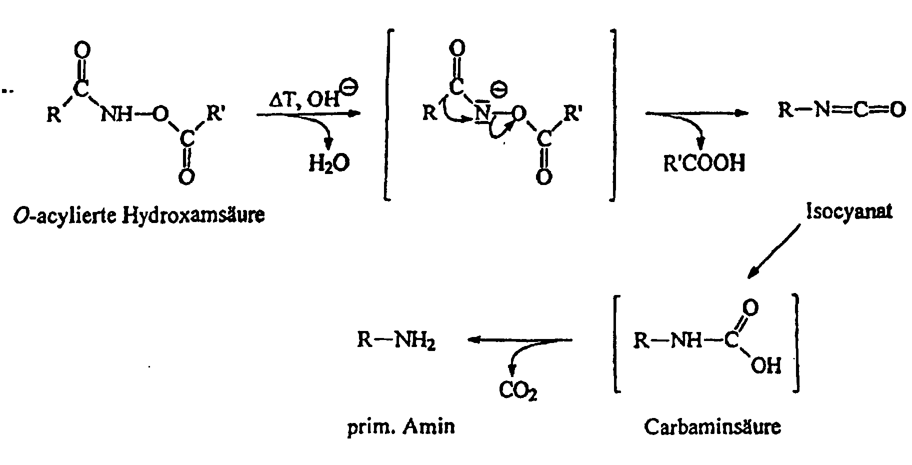

Als Basen sind Verbindungen, wie NaOH, KOH, NaNH₂, Na₂CO₃ geeignet, wobei die Art der verwendeten Basen von der Struktur der gewünschten Hydroxamsäure abhängt.

Zweckmäßige aprotische Lösungsmittel sind beispielsweise Toluol, Benzol oder Xylol.

Um die O-acylierte Hydroxamsäure möglichst quantitativ und rasch zu zersetzen, sind im allgemeinen Temperaturen von 100°C bis 150°C und vorzugsweise 100°C zweckmäßig.

Da bei der Lossen-Umlagerung die absolute Konfiguration des α-Kohlenstoffatoms der als Ausgangsmaterial eingesetzten optisch aktiven Hydroxamsäure erhalten bleibt (vgl. Campbell & Kenyon, 1946), sind die als Produkt der Lossen-Umlagerung erhaltenen primären Amine ebenfalls optisch aktiv.

Bei dem erfindungsgemäßen Verfahren zur Herstellung von optisch aktiven, primären Aminen ist es jedoch nicht erforderlich, das nichtumgesetzte Enantiomere der bei der Herstellung der optisch aktiven Hydroxamsäuren eingesetzten Amide, Carbonsäureester oder Carbonsäuren nach der ersten enzymkatalysierten Umsetzung zu entfernen, d.h. die reine, optisch aktive Hydroxamsäure einzusetzen. Die Umsetzung kann auch in der Weise geführt werden, daß man das Racemat aus den chiralen Amiden, Carbonsäureestern oder Carbonsäuren zunächst unter Enzymkatalyse mit Hydroxylamin umsetzt, und das dabei erhaltene Reaktionsgemisch, in dem neben der optisch aktiven Hydroxamsäure das nichtumgesetzte Enantiomere des Ausgangsmaterials enthalten ist, mit einem Carbodiimid unter Protonenkatalyse reagieren läßt, wodurch intermediär ein Isocyanat und ein entsprechendes Harnstoffderivat gebildet wird. Das Isocyanat reagiert dann sofort mit dem in dem Reaktionssystem enthaltenen Wasser zu einer Carbaminsäure, die ihrerseits zu Kohlendioxid und dem gewünschten primären, optisch aktiven Amin zerfällt. Das Amin kann dann von dem nichtumgesetzten Enantiomeren des Ausgangsmaterials in einfacher Weise getrennt werden.

Die Umsetzung mit Carbodiimid bietet den Vorteil, daß die in einem wäßrigen Reaktionssystem anfallende optisch aktive Hydroxamsäure gleich weiterverarbeitet werden kann und somit eine Reinigungsstufe unterbleiben kann. Die Derivatisierung mit einem Carbodiimid ist erforderlich, da die klassischen chemischen Methoden der O-Acylierung von Hydroxamsäure nur in aprotischen Lösungsmitteln durchgeführt werden können, diese jedoch für enzymkatalysierte Reaktionen unzweckmäßig sind.

Als solche Carbodiimide sind beispielsweise 1-Benzyl-3-(3'dimethylaminopropyl)carbodiimid, 1-Cyclohexyl-3-(2'-morpholinoethyl)carbodiimid und 1-Ethyl-3-(3'-dimethylaminopropyl)carbodiimid geeignet. Als besonders zweckmäßig erweist sich das 1-Ethyl-3-(3'-dimethylaminopropyl)carbodiimid (EDC). Dieses Carbodiimid ist beispielsweise in Sheehan, J.C., P.A. Cruickshank & G.L. Boshart, 1961, A convenient synthesis of water-soluble carbodiimides, J. Org. Chem. 26, 2525-2528, beschrieben.

Da bei der Herstellung der optisch aktiven Hydroxamsäure im allgemeinen ein Überschuß an Hydroxylamin eingesetzt wird, ist es zweckmäßig, das überschüssige Hydroxylamin vor der Umsetzung der Reaktionsmischung mit dem Carbodiimid zu entfernen. Hierzu wird das Reaktionsgemisch auf einen pH im Bereich von pH = 0 bis 2 und vorzugsweise im Bereich von pH = 1 angesäuert. Das Hydroxylamin zersetzt sich dann leicht zu Ammonium und Distickstoffmonoxid (vgl. Holleman, A.F. & E. Wiberg, 1985, Lehrbuch der Anorganischen Chemie, 91.-100. Auflage, S. 591, Walter de Gruyter Verlag, Berlin, New York).

Nach dem Ansäuern wird das Reaktionsgemisch auf eine Temperatur im Bereich von 40°C bis 100°C und vorzugsweise auf 80°C so lange erhitzt, bis keine Gasentwicklung mehr zu beobachten ist. Anschließend gibt man zu dem Reaktionsgemisch eine Base, wie beispielsweise Natronlauge, um den pH auf einen Wert von pH = 4 bis 6 und vorzugsweise auf pH = 5,0 einzustellen.

Anschließend erfolgt dann die Derivatisierung mit dem Carbodiimid gemäß dem folgenden allgemeinen Schema: 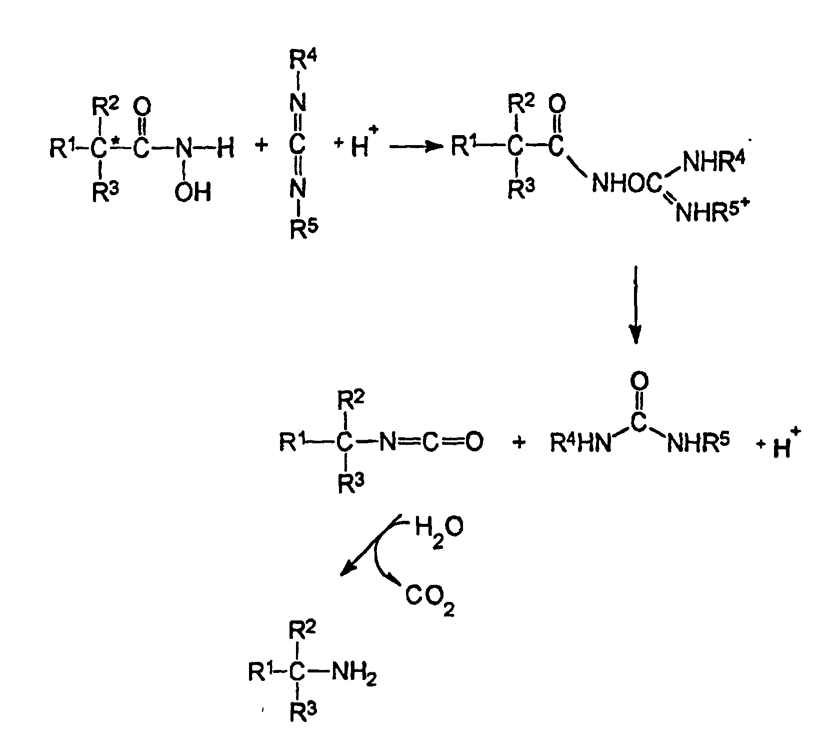 worin R¹ bis R⁵ wie voranstehend definiert sind.

Setzt man beispielsweise racemisches 2-Phenylpropionamid als Ausgangsmaterial bei dem erfindungsgemäßen Verfahren ein, so erhält man zunächst S-(-)-Phenylethylamin und R-2-Phenylpropionamid gemäß dem folgenden Formelschema:

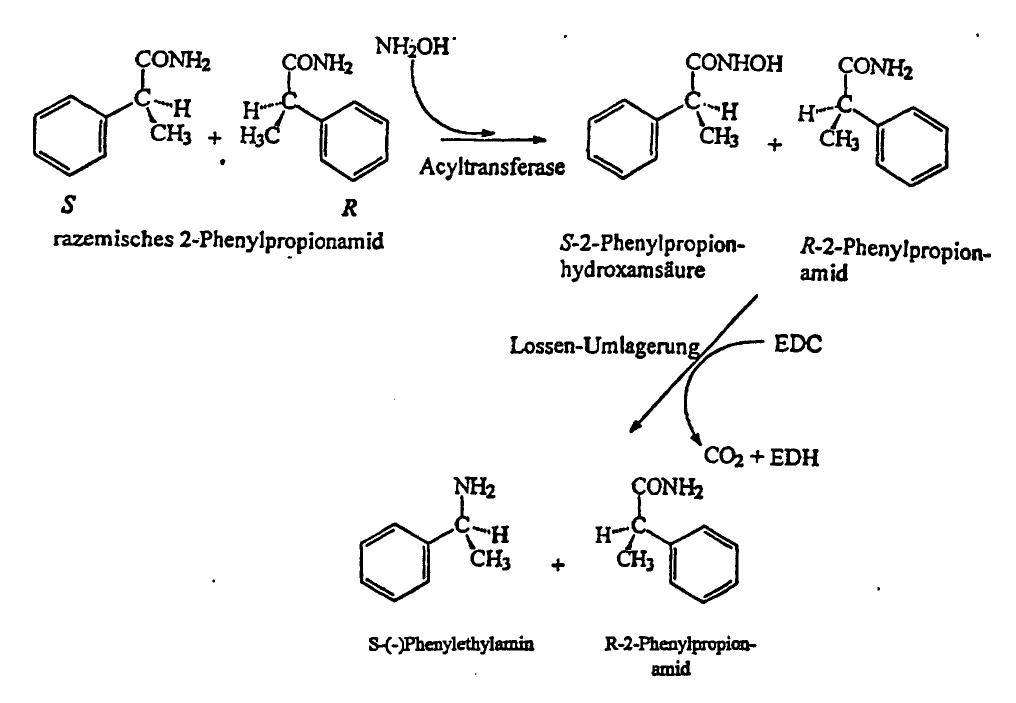

Das gewünschte optisch aktive, primäre Amin kann von dem nichtumgesetzten Enantiomeren des Ausgangsmaterials in einfacher Weise abgetrennt werden. Hierzu kann wie folgt vorgegangen werden: Die wäßrige Reaktionslösung wird mit Salzsäure auf pH = 1 angesäuert und mit Essigsäureethylester oder Diethylether oder Methylenchlorid extrahiert. Das Amid befindet sich nun in der organischen Phase. Danach wird der pH-Wert der wäßrigen Phase mit Natronlauge auf pH = 10 gebracht und das Amin mit den genannten Lösungsmitteln extrahiert.

Auf diese Weise können optisch aktive, primäre Amine wie S-(-)-Phenylethylamin, S-2-Aminobutan oder S-1-(1-Naphthyl)-ethylamin erhalten werden, die sonst nur durch aufwendige Racemattrennung zugänglich wären.

Die Erfindung wird durch die nachfolgenden Beispiele näher erläutert.

Zur Bestimmung der Hydroxamsäuren wurde dabei wie folgt vorgegangen:

Als zweizähnige Liganden bilden Hydroxamsäuren stabile, stark gefärbte Chelatkomplexe mit Eisen(III)-Ionen. Diese Trishydroxamatoeisen(III)-Komplexe [Fe(RCONHO)₃] haben ein Absorptionsmaximum bei 500 nm und erscheinen dem Auge tief dunkelrot'gefärbt. Diese Eigenschaft wird zur qualitativen (Buckles, R.E. & C.J. Thelen, 1950. Qualitative determination of carboxylic esters. Scope and limitations of hydroxamic acid test. Anal. Chem. 22, 676-678) und quantitativen (Brammar, W.J. & P.H. Clarke, 1964, Induction and repression of Pseudomonas aeruginosa amidase. J. Gen. Microbiol. 37, 307-319) Bestimmung von Hydroxamsäuren genutzt. Um den Eisenkomplex zu erhalten, mischt man eine hydroxamsäurehaltige Lösung mit einer salzsauren Eisen(III)chlorid-Lösung. Die Extinktion wird in einer Glasküvette bei 500 nm spektralphotometrisch bestimmt.

Eisen(III)chlorid-Lösung (modifiziert nach Hoare, D.G., A. Olson & D.E. Koshland JR., 1968. The reaction of hydroxamic acids with water-soluble carbodiimides. A Lossen rearrangement. J. Am. Chem. Soc. 90, 1638-1643):

| | |
|---|---|
| FeCl₃ x 6 H₂O | 13,51 g |
| konz. Salzsäure (12 M) | 3,33 ml |
| H₂O | ad 500 ml |

Die Lösung wurde durch ein Filter mit 0,45 µm Porengröße filtriert, um Trübungen zu entfernen.

Zu 300 µl einer hydroxamsäurehaltigen Probe [Hydroxamsäure, gelöst in Tris/HCl-Puffer (30 mM, pH 7,5)] pipettierte man 600 µl der salzsauren Eisen(III)chlorid-Lösung. Es bildete sich sofort ein tiefrot gefärbte Eisenkomplex. Die Extinktion wurde spektralphotometrisch bestimmt. Eichgeraden wurden mit Acethydroxamsäure und 2-Phenylpropionhydroxamsäure, gelöst in Tris/HCl-Puffer (30 mM, pH 7,5), gemessen. Diese zeigten eine lineare Abhängigkeit von Extinktion und Hydroxamatkonzentration im Bereich von 0,0 bis 1,5 mM Hydroxamsäure. Die molaren Extinktionskoeffizienten (ε_{λmax}500 nm) wurden für die Acethydroxamsäure zu 3500 lmol⁻¹ cm⁻¹, für Phenylacethydroxamsäure zu 3800 lmol⁻¹ cm⁻¹ und für die 2-Phenylpropionhydroxamsäure zu 4500 lmol⁻¹ cm⁻¹ bestimmt.

Enzymaktivitäten wurden sowohl mittels HPLC, als auch durch photometrische Methoden bestimmt. Die photometrischen Messungen erfolgten bei Raumtemperaturen in 1-ml-Glasküvetten mit einer Schichtdicke von 1 cm.

Die spezifische Aktivität wird in Enzymeinheiten pro mg Protein (U/mg) angegeben. Eine Unit ist die enzymatische Aktivität, die den Umsatz von 1 µmol Substrat bzw. die Bildung von 1 µmol Produkt in einer Minute katalysiert.

### Beispiel 1

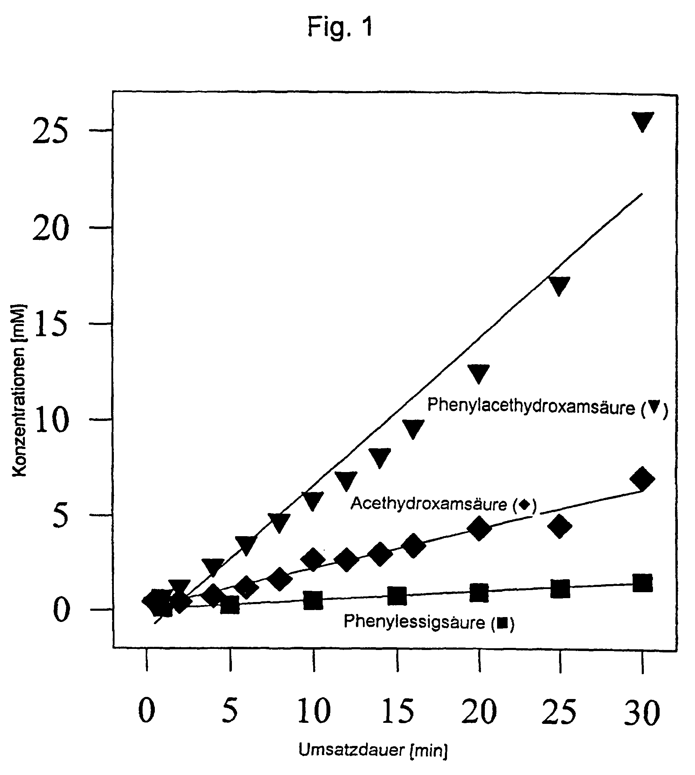

Zellen von Rhodococcus erythropolis MP50 wurden in ammoniumfreiem Mineralmedium mit Succinat (10 mM) als Kohlenstoff- und Energiequelle, Ketoprofenamid (1 mM) als Stickstoffquelle und 3 % Komplexmedium (NB) angezogen. Am Ende der exponentiellen Wachstumsphase wurden die Zellen durch Zentrifugation geerntet, in Tris/HCl-Puffer (30 mM, pH 7,5) einmal gewaschen und in demselben Puffer zu einer optischen Dichte (OD₅₄₆ₙₘ) von 16,0 resuspendiert. In zwei 25 ml-Erlenmeyerkolben mit Schikanen wurden je 2,75 ml Tris/HCl-Puffer (30 mM, pH 7,5) vorgelegt und mit 54 mg Phenylacetamid (PAA) oder 23,6 mg Acetamid versetzt. Die Kolben wurden anschließend bei 30°C in einem Schüttelwasserbad inkubiert, bis sich das PAA bzw. das Acetamid vollständig gelöst hatten. Dazu fügte man jeweils 1 ml einer frisch bereiteten und mit NaOH (10 M) frisch neutralisierten Hydroxylamin-Hydrochlorid-Lösung (2 M) hinzu. Der Umsatz wurde durch Zugabe von je 0,25 ml der Zellsuspension gestartet. Das Gesamtvolumen betrug 4 ml. Die optische Dichte(OD₅₄₆ₙₘ) im Versuch war 1,0. Der Ansatz enthielt 0,1 M Amid und 0,5 M Hydroxylamin. In Abständen von 2 min wurden je 0,3 ml der Zellsuspensionen entnommen und auf 600 µl einer salzsauren Eisen(III)chlorid-Lösung pipettiert. Die Zellen wurden durch Zentrifugation entfernt und der Überstand nochmals mit Eisen(III)chlorid-Lösung 1 zu 10 verdünnt. Die Extinktion wurde bei 500 nm gemessen und der Gehalt an Phenylacethydroxamsäure (▼) bzw. Acethydroxamsäure (◆) mittels der jeweiligen Eichgeraden bestimmt (vgl. Fig. 1). Parallel dazu wurden entsprechende Ansätze ohne Ruhezellen inkubiert, um die Entstehung von Hydroxamsäure durch eine chemische Reaktion auszuschließen. Im Beobachtungszeitraum von 30 min war keine Hydroxamsäurebildung in den Kontrollansätzen nachzuweisen.

In einem weiteren Ansatz (4 ml Gesamtvolumen, OD₅₄₆ₙₘ = 1,0) wurden Ruhezellen von Stamm MP50 mit PAA (100 mM) ohne Hydroxylamin inkubiert. Alle 5 min wurden 0,3 ml der Zellsuspension entnommen, die Zellen durch Zentrifugation entfernt und die Konzentration von Phenylessigsäure (■) mittels HPLC bestimmt.

Wie sich Fig. 1 entnehmen läßt, zeigt Rhodococcus erythropolis MP50 mit Acetamid und Phenylacetamid (PAA) als Substrat in Gegenwart von Hydroxylamin Acyltransferase-Aktivität, die deutlich höher liegt als die spezifische Aktivität der Zellen bei der Hydrolyse von PAA zur korrespondierenden Carbonsäure.

Die spezifischen Aktivitäten betrugen für die Bildung von PES bzw. Phenylacethydroxamsäure 0,54 bzw. 7,83 U/mg Protein. Die Acethydroxamsäure wurde mit einer spezifischen Aktivität von 2,22 U/mg Protein gebildet.

### Beispiel 2

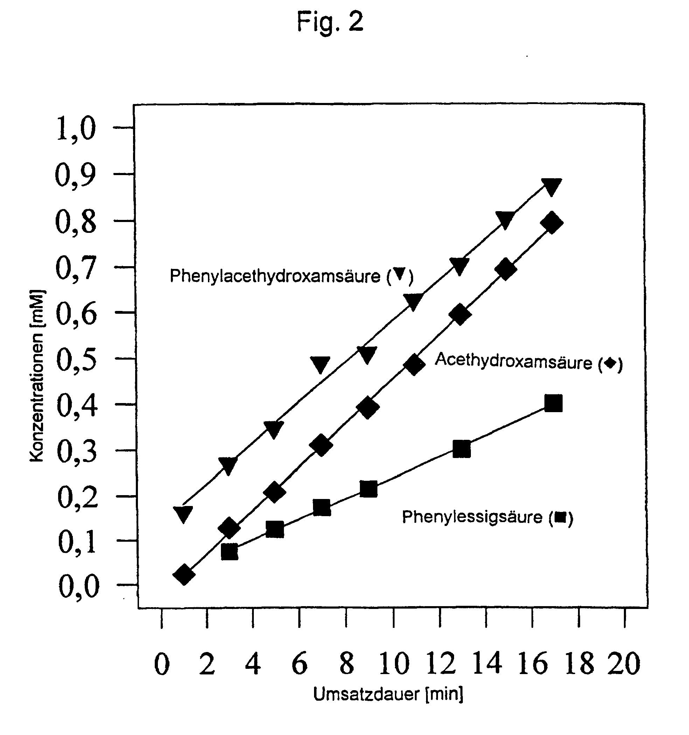

Nachdem Acyltransferase-Aktivität in ruhenden Zellen von Stamm MP50 in Beispiel 1 nachgewiesen werden konnte, wurden die Versuche mit Rohextrakten aus induzierten Zellen wiederholt. Es konnte ebenfalls in Gegenwart von Hydroxylamin Acyltransferase-Aktivität gegenüber PAA und Acetamid nachgewiesen werden (Fig. 2). Die enzymatische Hydrolyse von PAA zu PES erfolgte mit einer spezifischen Aktivität von 0,67 U/mg Protein. Die spezifischen Aktivitäten bei der Bildung von Phenylacethydroxamsäure bzw. Acethydroxamsäure waren 7,27 U/mg bzw. 7,80 U/mg Protein und damit um mehr als das Zehnfache höher als bei der Hydrolyse von PAA.

### Experimentell wurde dabei wie folgt vorgegangen:

Zellen von Rhodococcus erythropolis MP50 wurden mit Ketoprofenamid (1 mM) als Stickstoffquelle angezogen und in der späten exponentiellen Phase geerntet. Aus ihnen wurde Rohextrakt bereitet. 10 µl des Rohextraktes (6,5 µg Protein) wurden mit 640 µl Tris/HCl-Puffer (30 mM, pH 7,5) in einem Eppendorf-Reaktionsgefäß verdünnt und mit 250 µl einer frisch neutralisierten, wäßrigen Hydroxylaminhydrochloridlösung (2 M) gemischt. Die Umsetzung wurde durch Zugabe von 100 µl einer methanolischen Phenylacetamid-Lösung (PAA, 50 mM) oder einer Lösung von Acetamid (50 mM) in Tris/HCl-Puffer (30 mM, pH 7,5) gestartet und fand bei Raumtemperatur statt. Das Gesamtvolumen betrug 1 ml. Die Konzentrationen des Amids und des Hydroxylamins im Umsatz waren 5 mM und 0,5 M. Alle zwei Minuten wurden 100 µl des Ansatzes entnommen und mit 800 µl einer salzsauren Eisen(III)chlorid-Lösung gemischt. Die stark salzsaure Lösung denaturierte das Protein und beendete die enzymatische Reaktion. Ausgefälltes Protein wurde abzentrifugiert und die Extinktion des Überstandes bei 500 nm spektralphotometrisch bestimmt. Die Bestimmung der Konzentration von Phenylacethydroxamsäure (▼) bzw. Acethydroxamsäure (◆) erfolgte mit entsprechenden Eichgeraden. Zur Bestimmung der Amidase-Aktivität wurden 10 µl Rohextrakt in 0,89 ml Tris/HCl-Puffer (30 mM, pH 7,5) verdünnt. Die Umsetzung wurde durch Zugabe von 100 µl methanolischer PAA-Lösung gestartet. Das Gesamtvolumen betrug 1 ml und die PAA-Konzentration war 5 mM. In regelmäßigen Zeitabständen wurden je 100 µl des Reaktionsgemisches auf 10 µl 1 N HCl pipettiert, ausgefälltes Protein abzentrifugiert und die PES-Konzentration (■) mittels HPLC bestimmt.

### Beispiel 3

Eine Acyltransferase-Aktivität konnte in Ruhezellen und in Rohextrakt von Stamm MP50 nachgewiesen werden. Es blieb noch zu zeigen, ob tatsächlich die Amidase- und Acyltransferase-Aktivität von demselben Enzym herrührten. Die Hydroxamsäurebildung mit zur Homogenität gereinigter Amidase bewies, daß es sich bei Acyltransferase und Amidase um ein und dasselbe Enzym handelte (Tab. 1).

Wie die nachstehende Tabelle 1 zeigt, war die Acyltransferase-Aktivität gegenüber 2-Phenylpropionamid rund dreimal so hoch wie die Amidase-Aktivität. Die Bildung von Acethydroxamsäure verlief viermal so rasch wie die Verseifung zur Essigsäure und die Phenylacethydroxamsäure entstand sogar neunmal rascher als Phenylessigsäure. Die Amidase aus Stamm MP50 zeigte also durchweg bei der Hydroxamsäurebildung höhere spezifische Aktivitäten als bei der Hydrolyse der Amide zu den Carbonsäuren.

**Tabelle 1**

| Spezifische Aktivitäten bei der Bildung von Carbonsäuren und Hydroxamsäuren aus Carbonsäureamiden durch die gereinigte Amidase | | |
|---|---|---|
| Substrat | Produkt | spez. Aktivität [U/mg Protein] |
| Acetamid | Essigsäure | 0,83 |
| Acetamid | Acethydroxamsäure | 3,54 |
| Phenylacetamid | Phenylessigsäure | 4,10 |
| Phenylacetamid | Phenylacethydroxamsäure | 36,61 |
| 2-Phenylpropionamid | 2-Phenylpropionsäure | 4,50 |
| 2-Phenylpropionamid | 2-Phenylpropionhydroxamsäure | 13,02 |

Zur Bestimmung der Acyltransferase-Aktivität wurden in einem Eppendorf-Reaktionsgefäß 10 µl gereinigte Amidase (2,6 µg Protein) mit 0,64 ml Na-Phosphatpuffer (10 mM, pH 7,5) verdünnt. Dazu wurden 0,25 ml einer frisch zubereiteten und mit Natronlauge neutralisierten Hydroxylaminhydrochloridlösung (2 M) pipettiert. Der Umsatz wurde durch Zugabe von 0,1 ml einer methanolischen Stammlösung (50 mM) des jeweiligen Amids gestartet. Acetamid (50 mM) wurde in Na-Phosphatpuffer (10 mM, pH 7,5) gelöst und dann zugegeben. Das Gesamtvolumen betrug 1 ml. Die Konzentrationen von Hydroxylamin und dem jeweiligen Amid waren 0,5 M bzw. 5 mM. In einem Zeitraum von 20 min wurden viermal je 0,1 ml des Reaktionsgemischs auf 0,8 ml salzsaurer Eisen(III)chlorid-Lösung pipettiert und die Extinktion des Hydroxamsäure-Komplexes photometrisch bei 500 nm gemessen. Die Konzentration der Hydroxamsäuren wurde über entsprechende Eichgeraden ermittelt.

Zur Bestimmung der Amidase-Aktivität wurde dieselbe Proteinmenge in 0,89 ml Na-Phosphatpuffer (10 mM, pH 7,5) verdünnt und dann mit dem jeweiligen Amid (5 mM) versetzt. Die Konzentrationen der gebildeten Carbonsäuren wurden mittels HPLC gemessen. Beim Umsatz von Acetamid wurde die Menge an freigesetztem Ammonium durch Indophenol-Methode bestimmt.

### Beispiel 4

Der Nachweis, daß die Acyltransferasen und insbesondere die aus Rhodococcus erythropolis MP50 gewonnene Acyltransferase als enantioselektiver Katalysator wirkt, wurde wie folgt geführt:

Als Modellsubstrat für die Untersuchungen wurde racemisches 2-Phenylpropionamid (2-PPA) gewählt. Das gereinigte Enzym wurde mit (R,S)-2-PPA (5 mM) in Gegenwart von Hydroxylamin (0,5 M) inkubiert. Die Hydroxamsäurebildung wurde dabei sowohl photometrisch als auch durch Ionenpaar-Chromatographie verfolgt.

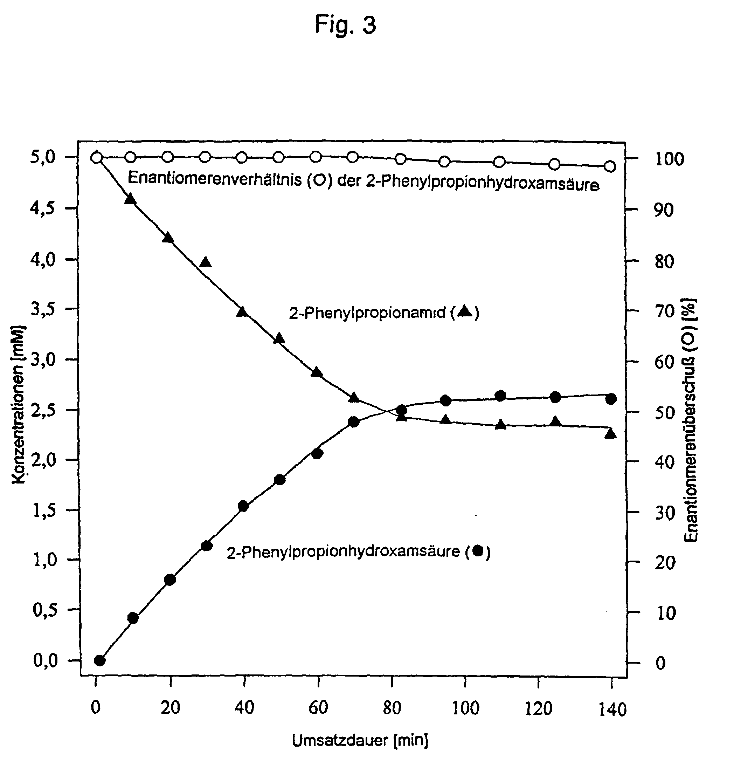

Die Versuche ergaben, daß sich die Hydroxamsäurebildung nach 50%igem Umsatz des Substrats drastisch verlangsamte (Fig. 3). Die Untersuchung der enzymatisch gebildeten 2-Phenylpropionhydroxamsäure (2-PPHS) mittels einer chiralen HPLC-Säule zeigte, daß bis zu einem Umsatz von 46 % des Substrats nur ein Enantiomeres der Hydroxamsäure entstanden war. Die Bildung von 2-PPS war in Anwesenheit von Hydroxylamin nicht zu beobachten. Tatsächlich war die Amidase aus Stamm MP50 in der Lage, racemisches 2-PPA enantioselektiv zu 2-PPHS umzusetzen.

Die spezifische Aktivität zu Beginn der Umsetzung betrug 9,73 U/mg Protein für die Hydroxamsäurebildung und lag damit etwas unter dem maximal erreichten Wert von 13,02 U/mg (vgl. Tab. 1). Sehr deutlich war die ausgeprägte Abnahme der Aktivität zu sehen, wenn 50 % des racemischen Substrats umgesetzt waren.

Die Trennung der enzymatisch gebildeten Hydroxamsäure durch eine chirale HPLC-Säule zeigte, daß bis zu einem Umsatz von 46 % nur ein Enantiomeres auftrat. Der Enantiomerenüberschuß lag also über 99 %.

### Experimentell wurde dabei wie folgt vorgegangen:

10 µl gereinigte Amidase (2,6 µg Protein) wurden in einem Eppendorf-Reaktionsgefäß in 0,64 ml Na-Phosphatpuffer (10 mM, pH 7,5) verdünnt und dann bei Raumtemperatur mit 0,25 ml einer frisch bereiteten und neutralisierten Hydroxylaminhydrochlorid-Lösung (2 M) 10 min lang inkubiert. Die Umsetzung wurde durch Zugabe von 0,1 ml einer methanolischen Lösung von (R,S)-2-PPA (50 mM Stammlösung) gestartet. Das Gesamtvolumen betrug 1 ml. Die Konzentration von Hydroxylamin war 0,5 M. Die Amidkonzentration betrug zu Beginn 5 mM. In regelmäßigen Zeitabständen wurden jeweils 50 µl des Reaktionsgemischs auf 5 µl 1 N HCl pipettiert. Denaturiertes Protein wurde abzentrifugiert und die Amid- (▼) bzw. Hydroxamsäurekonzentration (•) durch Ionenpaar-Chromatgraphie bestimmt. Das Enantiomerenverhältnis (○) der 2-PPHS wurde mit einer chiralen HPLC-Säule (Chiral-HSA) untersucht. Dazu mußten 2-PPA und 2-PPHS voher durch eine Grom-Sil 120 TMS-2CP-Fertigsäule getrennt werden.

### Beispiel 5

Zur Umsetzung der in Beispiel 4 hergestellten optisch reinen 2-Phenylpropionhydroxamsäure in das entsprechende ebenfalls optisch reine 1-Phenylethylamin wurde wie folgt vorgegangen:

Zur Entfernung des überschüssigen Hydroxylamins wurde das in Beispiel 4 erhaltene Reaktionsprodukt zunächst mit 1 N HCl angesäuert. Danach betrug der pH-Wert der Lösung etwa 0 bis 1. Durch das Ansäuern zersetzte sich das Hydroxylamin zu Ammonium und Distickstoffmonoxid. Diese Zersetzung wurde dadurch gefördert, daß das Reaktionsgemisch für 10 min in einem Wasserbad bei 80°C gehalten wurde. Nach Beendigung der Gasentwicklung wurde der pH-Wert wieder mit Natronlauge auf etwa 5 eingestellt.

Anschließend wurde die etwa 10fache Menge an 1-Ethyl-3-(3'dimethylaminopropyl)carbodiimid (EDC) - bezogen auf die 2-Phenylpropionhydroxamsäure - zugegeben.

Die Konzentration des gebildeten 1-Phenylethylamins wurde durch Ionenpaar-Chromatographie bestimmt und sein Enantiomerenüberschuß mit Hilfe einer chiralen HPLC-Säule (Crownpak CR(+)) ermittelt.

### Beispiel 6

Mit denselben Reaktanten, wie in Beispiel 5 beschrieben, wurde eine Umsetzung durchgeführt, wobei jedoch für jeden Zeitpunkt, an dem die enzymatische Hydroxamsäurebildung abgestoppt werden sollte, ein eigener Ansatz gestartet wurde. Dazu wurden 10 µl der gereinigten Amidase (1,7 x 10⁻³ mg Protein) mit 315 µl Na-Phosphatpuffer (54 mM, pH 7,5) in einem Eppendorf-Reaktiongefäß verdünnt und mit 125 µl einer frisch bereiteten und neutralisierten Hydroxylaminhydrochlorid-Lösung (2 M) inkubiert. Die enzymatische Reaktion wurde durch Zugabe von 50 µl einer methanolischen Lösung von 2-PPA (50 mM) gestartet. Das Gesamtvolumen jedes Ansatzes betrug 0,5 ml. Die Konzentrationen von 2-PPA und Hydroxylamin waren 5 mM und 0,5 M. Es wurden 10 identische Ansätze gestartet und in Abständen von jeweils 10 min durch die Zugabe von 50 µl 1 N HCl gestoppt. Ausgefälltes Protein wurde durch Zentrifugation entfernt und aus dem Ansatz wurden 50 µl für HPLC-Messungen zur Konzentrationsbestimmung von 2-PPA und 2-PPHS entnommen. Die verbliebenen 500 µl wurden zur Verkochung des Hydroxylamins in einem Wasserbad 10 min lang auf 80°C erhitzt. Nach Abkühlen der Proben auf Raumtemperatur wurde der pH-Wert durch Zugabe von 29 µl 1 N NaOH auf 4,5 eingestellt. Dazu gab man 9,6 mg EDC (100 mM) und erhitzte erneut für eine Stunde auf 80°C. Die Konzentration des gebildeten 1-Phenylethylamins wurde wie in Beispiel 3 durch Ionenpaar-Chromatographie bestimmt und sein Enantiomerenüberschuß mit Hilfe einer chiralen HPLC-Säule (Crownpak CR(+)) ermittelt.

### Beispiel 7

Zur Herstellung größerer Mengen an Hydroxamsäure wird zunächst zur Gewinnung des enzymatisch aktiven Materials wie folgt vorgegangen:

Zunächst wird ein Kulturmedium für die Anzucht von Rhodococcus erythropolis MP50 mit der folgenden Zusammensetzung hergestellt:

| | |
|---|---|
| NaHPO₄ x 12 H₂O | 14,0 g |
| KH₂PO₄ | 2,0 g |
| CaCl₂ x 2 H₂O | 0,005 g |
| Fe(III)citrat x 7 H₂O | 0,02 g |
| MgSO₄ x 7 H₂O | 1,0 g |
| Spurenelementlösung SL6 (nach Pfennig & Lipper, 1966) aber ohne EDTA und FeSO₄ | 1,0 ml |
| Dinatriumsuccinat | 1,62 g |
| Nutrient Broth-Lösung | 30 ml |
| Phenylacetonitril | 0,18 g |
| H₂O | ad 1000 ml |

10 ml dieses Mediums wurden mit 0,1 ml einer Flüssigkultur von Rhodococcus erythropolis MP50 auf Nutrient Broth angeimpft und über Nach in einem Erlenmeyerkolben mit Schikanen bei 30°C auf einem Schüttler bei 150 U/min inkubiert. Diese Kultur diente zum Animpfen von 100 ml frischen Mediums in einem Erlenmeyerkolben mit Schikanen, der unter denselben Bedingungen bei 18 bis 24 h inkubiert wurde. Diese Kultur wiederum diente als Inokulum für 700 ml Kulturmedium in einem 3 l-Erlenmeyerkolben mit Kerben. Die Zellen wurden in der späten exponentiellen Wachstumsphase durch Zentrifugation geerntet, verwendet oder bei -70°C schockgefroren und bei -25°C gelagert. Zellaufschluß, Gewinnung von Rohextrakt und Enzymreinigung erfolgt, wie in Journal of Bacteriology beschrieben. B. Hirrlinger, A. Stolz & H.-J. Knackmuss (1996) Purification and Properties of an Amidase from Rhodococcus erythropolis MP50 which Enantioselectively Hydrolyzes 2-Arylpropionamides, Journal of Bacteriology 178 (12), S. 3501-3507.

Mit dem so gewonnenen enzymatisch aktiven Material wurde anschließend S-2-Phenylpropionhydroxamsäure wie folgt hergestellt:

125 U Acyltransferase wurden in Form ganzer Zellen, Rohextrakt oder gereinigtem Enzym mit 3,2 l Na-Phosphatpuffer (10 mM, pH 7,5) in einer Weithals-Schottflasche mit 10 l Volumen mit Hilfe eines Magnetrührers vermischt. Dazu wurden 1,25 l einer frisch bereiteten und mit Natronlauge auf pH = 7 eingestellten wäßrigen Hydroxylaminhydrochlorid-Lösung (2 M) hinzugefügt und 10 min bei Raumtemperatur gerührt. Die enzymatische Reaktion wurde durch die Zugabe von 3,725 g (0,025 mol) (R,S)-Phenylpropionamid (gelöst in 500 ml Methanol) gestartet. Der Reaktionsverlauf wurde mittels Ionenpaar-Chromatographie verfolgt. Nach 120 min war der Umsatz der S-Enantiomeren des Amids zur korrespondierenden Hydroxamsäure beendet und die Zellen wurden durch Zentrifugation entfernt, bzw. der Rohextrakt oder das gereinigte Enzym durch Erhöhung des pH-Wertes mit Natronlauge auf 10 denaturiert und dann abzentrifugiert.

Zur Trennung der enzymatisch gebildeten S-2-Phenylpropionhydroxamsäure von nicht umgesetztem R-2-Phenylpropionamid wurde das Amid durch Ausschütteln mit Essigsäureethylester oder Methylenchlorid aus der alkalischen wäßrigen Lösung abgetrennt. Die wäßrige Lösung wurde anschließend mit konz. HCl auf pH = 2 eingestellt und erneut mit Essigsäureethylester oder Methylenchlorid extrahiert. Nach Entfernen des organischen Lösungsmittels im Vakuum erhielt man 1,9 g (0,012 mol) S-2-Phenylpropionhydroxamsäure. Der Enantiomerenüberschuß, der mittels Messungen mit einer chiralen HPLC-Säule bestimmt wurde war >99 %.

### Beispiel 8

Mit dem, wie in Beispiel 7 beschrieben, hergestellten enzymatisch aktiven Material wurde S-2-Phenylpropionhydroxamsäure wie folgt hergestellt:

125 U Acyltransferase wurden in Form immobilisierter ganzer Zellen oder immoblisiertem gereinigtem Enzym mit 3,2 l Na-Phosphatpuffer (10 mM, pH 7,5) in einer Weithals-Schottflasche mit 10 l Volumen mit Hilfe eines Magnetrührers vermischt. Dazu wurden 1,25 l einer frisch bereiteten und mit Natronlauge auf pH = 7 eingestellten wäßrigen Hydroxylamin-Hydrochlorid-Lösung (2 M) hinzugefügt und 10 min bei Raumtemperatur gerührt. Die enzymatische Reaktion wurde durch die Zugabe von 3,725 g (0,025 mol) (R,S)-2-Phenylpropionamid (gelöst in 500 ml Methanol) gestartet. Der Reaktionsverlauf wurde mittels Ionenpaar-Chromatographie verfolgt. Nach 120 min war der Umsatz des S-Enantiomeren des Amids zur korrespondierenden Hydroxamsäure beendet und die immobilisierten Zellen bzw. das immobilisierte Enzym wurden durch Filtration abgetrennt und für erneute Verwendung in Na-Phosphatpuffer (10 mM, pH 7,5) bei +4°C gelagert.

Zur Trennung der enzymatisch gebildeten S-2-Phenylpropionhydroxamsäure von nicht umgesetztem R-2-Phenylpropionamid wurde die wäßrige Lösung mit Natronlauge auf pH = 10 eingestellt und das Amid durch Ausschütteln mit Essigsäureethylester oder Methylenchlorid aus der alkalischen wäßrigen Lösung abgetrennt. Die wäßrige Lösung wurde anschließend mit konz. HCl auf pH = 2 eingestellt und erneut mit Essigsäureethylester oder Methylenchlorid extrahiert. Nach Entfernen des organischen Lösungsmittels im Vakuum erhielt man 1,9 g (0,012 mol) S-2-Phenylpropionhydroxamsäure. Der Enantiomerenüberschuß, der mittels Messungen mit einer chiralen HPLC-Säule bestimmt wurde, war >99 %.

### Beispiel 9

Die nach den in den Beispielen 7 bis 9 beschriebenen Verfahren gewonnenen enantiomerenreinen Hydroxamsäuren können nach O-Acylierung durch Erhitzen oder durch die Behandlung mit Basen in aprotischen Lösungsmitteln in das Isocyanat überführt werden. Nach Zugabe von Wasser bildet das Isocyanat eine instabile Carbaminsäure, die unter CO₂-Abgabe zum primären Amin weiterreagiert. Da die Lossen-Umlagerung unter Konfigurationserhalt am wandernden Kohlenstoffatom verläuft, erhält man als Produkt optisch aktive primäre Amine.

### Beispiel 10

Zur Erzeugung chiraler primärer Amine ohne vorherige Isolation der Hydroxamsäure wurde wie folgt vorgegangen:

125 U Acyltransferase wurden in Form ganzer Zellen, Rohextrakt oder gereinigtem Enzym mit 3,2.l Na-Phosphatpuffer (10 mM, pH 7,5) in einer Weithals-Schottflasche mit 10 l Volumen mit Hilfe eines Magnetrührers vermischt. Dazu wurden 1,25 l einer frisch bereiteten und mit Natronlauge auf pH 7 eingestellten wäßrigen Hydroxylamin-Hydrochlorid-Lösung (2 M) hinzugefügt und 10 min bei Raumtemperatur gerührt. Die enzymatische Reaktion wurde durch die Zugabe von 3,725 g (0,025 mol) (R,S)-2-Phenylpropionamid (gelöst in 500 ml Methanol) gestartet. Der Reaktionsverlauf wurde mittels Ionenpaar-Chromatographie verfolgt. Nach 120 min war der Umsatz des S-Enantiomeren des Amids zur korrespondierenden Hydroxamsäure beendet und die Zellen wurden durch Zentrifugation entfernt, bzw. der Rohextrakt oder das gereinigte Enzym durch Ansäuern mit konz. HCl auf pH = 1 denaturiert und dann abzentrifugiert.

Zur Entfernung des überschüssigen Hydröxylamins wurde die wäßrge Lösung unter Rühren für 30 min auf 80°C erwärmt. Danach war keine Gasentwicklung durch zerfallendes Hydroxylamin mehr zu beobachten. Nach Abkühlen der Lösung auf Raumtemperatur wurde ihr pH-Wert mit Natronlauge auf pH = 4,5 eingestellt. Zur Derivatisierung der Hydroxamsäure wurden 96 g (0,62 mol) 1-Ethyl-3-(3'-dimethylaminopropyl)carbodiimid (EDC) unter Rühren zu der wäßrigen Lösung gegeben und die Lösung danach unter Rühren für 2 h auf 80°C erhitzt.

Zur Trennung des aus der enzymatischen Reaktion verbliebenen R-2-Phenylpropionamids von dem bei der Lossen-Umlagerung entstandenen S-1-Phenylethylamins wurde die wäßrige Lösung mit konz. HCl auf pH = 1 angesäuert und das Amid mit Essigsäureethylester oder Methylenchlorid ausgeschüttelt. Die wäßrige Lösung wurde danach mit Natronlauge auf pH = 10 eingestellt und das Amin durch Ausschütteln mit Essigsäureethylester oder Methylenchlorid isoliert. Nach Entfernen des Lösungsmittels im Vakuum erhielt man 1,2 g (0,096 mol) S-1-Phenylethylamin mit einem Enantiomerenüberschuß > 99 %.

### Beispiel 11

Analog zu der in Beispiel 10 beschriebenen Vorgehensweise wurde S-1-Phenylethylamin wie folgt ohne vorherige Isolation der entsprechenden Hydroxamsäure durch Lossen-Umlagerung wie folgt hergestellt:

125 U Acyltransferase wurden in Form immobilisierter ganzer Zellen oder immobilisiertem gereinigtem Enzym mit 3,2 l Na-Phosphatpuffer (10 mM, pH 7,5) in einer Weithals-Schottflasche mit 10 l Volumen mit Hilfe eines Magnetrührers vermischt. Dazu wurden 1,25 l einer frisch bereiteten und mit Natronlauge auf pH = 7 eingestellten wäßrigen Hydroxylamin-Hydrochlorid-Lösung (2 M) hinzugefügt und 10 min bei Raumtemperatur gerührt. Die enzymatische Reaktion wurde durch die Zugabe von 3,725 g (0,025 mol) (R,S)-2-Phenylpropionamid (gelöst in 500 ml Methanol) gestartet. Der Reaktionsverlauf wurde mittels Ionenpaar-Chromatographie verfolgt. Nach 120 min war der Umsatz des S-Enantiomeren des Amids zur korrespondierenden Hydroxamsäure beendet und die immobilisierten Zellen bzw. das immobilisierte Enzym wurden durch Filtration abgetrennt und für erneute Verwendung in Na-Phosphatpuffer (10 mM, pH 7,5) bei +4°C gelagert.

Zur Entfernung des überschüssigen Hydroxylamins wurde die wäßrige Lösung mit konz. HCl auf pH = 1 eingestellt und unter Rühren für 30 min auf 80°C erwärmt. Danach war keine Gasentwickung durch zerfallendes Hydroxylamin mehr zu beobachten. Nach Abkühlen der Lösung auf Raumtemperatur wurde ihr pH-Wert mit Natronlauge auf pH = 4,5 eingestellt. Zur Derivatisierung der Hydroxamsäure wurden 96 g (0,62 mol) 1-Ethyl-3-(3'-dimethylaminopropyl)carbodiimid (EDC) unter Rühren zu der wäßrigen Lösung gegeben und die Lösung danach durch Rühren für 2 h auf 80°C erhitzt.

Zur Trennung des aus der enzymatischen Reaktion verbliebenen R-2-Phenylpropionamids von dem bei der Lossen-Umlagerung entstandenen S-1-Phenylethylamins wurde die wäßrige Lösung mit konz. HCl auf pH = 1 angesäuert und das Amid mit Essigsäureethylester oder Methylenchlorid ausgeschüttelt. Die wäßrige Lösung wurde danach mit Natronlauge auf pH = 10 eingestellt und das Amin durch Ausschütteln mit Essigsäureethylester oder Methylenchlorid isoliert. Nach Entfernen des Lösungsmittels im Vakuum erhielt man 1,2 g (0,096 mol) S-1-Phenylethylamin mit einem Enantiomerenüberschuß > 99 %.

## Patentansprüche

1. Verfahren zur Herstellung optisch aktiver Hydroxamsäuren der allgemeinen Formel 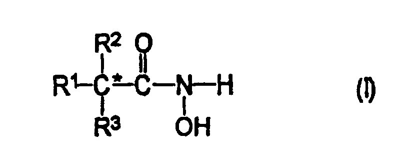 worin R¹, R² und R³ verschieden sind und für einen cyclischen oder linearen, aliphatischen oder aromatischen, substituierten oder unsubstituierten Kohlenwasserstoffrest, der ggf. Heteroatome enthalten kann, stehen, **dadurch gekennzeichnet, daß** man ein Racemat aus chiralen Amiden, Carbonsäureestern oder Carbonsäuren der allgemeinen Formel: 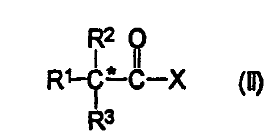 worin R¹, R² und R³ wie oben definiert sind und X für -NH₂, -OR oder -OH steht, wobei R ein beliebiger organischer Rest ist,
mit Hydroxylamin NH₂OH in Gegenwart einer Acyltransferase umsetzt und anschließend die gebildete optisch aktive Hydroxamsäure (I) von dem nichtumgesetzten Enantiomeren der allgemeinen Formel (II) abtrennt.

2. Verfahren nach Anspruch 1, **dadurch gekennzeichnet, daß** man als Acyltransferase eine Acyltransferase aus den Mikroorganismen der Gruppe Mycobacteriaceae, Mycobacterum smegmatis, Pseudomonas aeruginosa, Arthrobacter, Aspergillus nidulans, Bradyrhizobium japonicum, Brevibacterium sp. R312. Methylophilus methylotrophus, Pseudomonas chlororaphis, Pseudomonas fluorescens, Rhodococcus rhodochrous J1 und Rhodococcus erythropolis MP50 verwendet.

3. Verfahren nach Anspruch 2, **dadurch gekennzeichnet, daß** man als Acyltransferase die Acyltransferase aus Rhodococcus erythropolis MP50 verwendet.

4. Verfahren nach Anspruch 2 oder 3, **dadurch gekennzeichnet, daß** man die Acyltransferase in Form eines Rohextrakts aus den Mikroorganismen einsetzt.

5. Verfahren nach Anspruch 2 oder 3, **dadurch gekennzeichnet, daß** man die Acyltransferase in gereinigter Form einsetzt.

6. Verfahren nach den Ansprüchen 1 bis 5, **dadurch gekennzeichnet, daß** man die Umsetzung in Gegenwart eines Puffers bei einem pH-Wert von 6,5 bis 7,5 durchführt.

7. Verfahren nach den Ansprüchen 1 bis 6, **dadurch gekennzeichnet, daß** man die Reste R¹, R² und R³ aus der Gruppe, bestehend aus Methyl, Ethyl, n-Propyl, i-Propyl, n-Butyl, i-Butyl, t-Butyl, n-Pentyl, Cyclohexyl, Phenyl, t-Butoxyphenyl, Naphthyl, 3-Benzoylphenyl, Amino, Hydroxy, auswählt.

8. Verfahren nach Anspruch 7, **dadurch gekennzeichnet, daß** man als Verbindung der Formel (I) Alkylaminopropionsäure, p-Butoxyphenylessigsäure, 2-Phenylpropionsäure, 2-Methylbutansäure, 2-(1-Naphthyl)-propionsaure, 2-(6-Methoxy-2-naphthyl)propionsäure (Naproxen), 2-(3'-Benzoylphenyl)propionsäure (Ketoprofen) und deren Ester oder Amide verwendet.

9. Verfahren nach den Ansprüchen 1 bis 8, **dadurch gekennzeichnet, daß** man als Verbindung der allgemeinen Formel (I) ein Amid verwendet.

10. Verfahren nach den Ansprüchen 1 bis 9, **dadurch gekennzeichnet, daß** man die Verbindung der allgemeinen Formel (I) mit der 2- bis 10fachen Menge, vorzugsweise der 5fachen Menge, an Hydroxylamin bei einer Acyltransferaseaktivität von 3000 bis 5000 Units pro mol Substrat, vorzugsweise von 5000 Units pro mol Substrat, umsetzt.

11. Verfahren zur Herstellung von optisch aktiven primären Aminen der allgemeinen Formel: 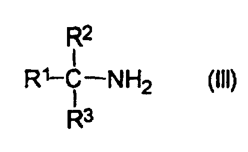 worin R¹, R² und R³ wie in Anspruch 1 definiert sind, **dadurch gekennzeichnet, daß** man die bei dem Verfahren nach den Ansprüchen 1 bis 10 erhaltene optisch aktive Hydroxamsäure der allgemeinen Formel (I) O-acyliert und die O-acylierte Hydroxamsäure durch Erhitzen oder durch die Behandlung mit Basen in aprotischen Lösungsmitteln zum entsprechenden Isocyanat umsetzt und das Isocyanat mit Wasser unter Abspaltung von CO₂ zum Amin der allgemeinen Formel (III) reagieren läßt.

12. Verfahren zur Herstellung von optisch aktiven primären Aminen der allgemeinen Formel: 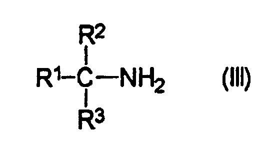 worin R¹, R² und R³ wie in Anspruch 1 definiert sind, **dadurch gekennzeichnet, daß** man die bei dem Verfahren nach den Ansprüchen 1 bis 10 erhaltene optisch aktive Hydroxamsäure der allgemeinen Formel (I) vor ihrer Abtrennung von dem nichtumgesetzten Enantiomeren der allgemeinen Formel (II) mit einem Carbodiimid der allgemeinen Formel 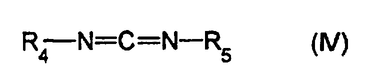 worin R₄ für Benzyl, Cyclohexyl oder Ethyl und R₅ Dimethylaminopropyl oder Morpholinoethyl stehen, umsetzt und anschließend das gebildete optisch aktive primäre Amin der allgemeinen Formel (III) von dem nichtumgesetzten Enantiomeren der allgemeinen Formel (II) abtrennt.

13. Verfahren nach Anspruch 12, **dadurch gekennzeichnet, daß** man das die optisch aktive Hydroxamsäure (I) und das nichtumgesetzte Enantiomere (II) enthaltende Reaktionsgemisch vor seiner Umsetzung mit dem Carbodiimid auf einen pH-Wert von 0 bis 2, vorzugsweise auf einen pH-Wert von 1, ansäuert, das Reaktionsgemisch anschließend auf eine Temperatur von 40°C bis 100°C, vorzugsweise 80°C, erhitzt und die Umsetzung mit dem Carbodiimid nachfolgend bei einem pH-Wert von 4 bis 6, vorzugsweise einem pH-Wert von 5,0, durchführt.

14. Verfahren nach Anspruch 12 oder 13, **dadurch gekennzeichnet, daß** man als Carbodiimid 1-Ethyl-3-(3'-dimethylaminopropyl)carbodiimid verwendet.

## Claims

1. Method of producing optically active hydroxamic acids of the general formula 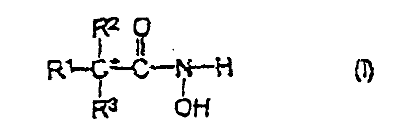 in which R¹, R² and R³ are different and can stand for a cyclic or linear, aliphatic or aromatic, substituted or non-substituted hydrocarbon residue, which can possibly contain heteroatoms, **characterised in that** a racemate of chiral amides, carbonic esters or carboxylic acids of the general formula: 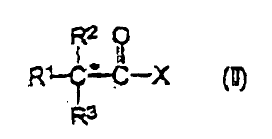 in which R¹, R² and R³ are as defined above and X stands for -NH₂, -OR or -OH, R being any organic residue,
is converted with hydroxylamine NH₂OH in the presence of an acyl transferase and then the optically active hydroxamic acid (I) formed is separated from the non-converted enantiomers of the general formula (II).

2. Method according to claim 1, **characterised in that**, as the acyl transferase is used an acyl transferase from the microorganisms of the group mycobacteriaceae, mycobacterium smegmatis, pseudomonas aeruginosa, arthrobacter, aspergillus nidulans, bradyrhizobium japonicum, brevibacterium sp. R312, methylophilus methylotrophus, pseudomonas chlororaphis, pseudomonas fluorescens, rhodococcus rhodochrous J1 and rhodococcus erythropolis MP50.

3. Method according to claim 2, **characterised in that** that the acyl transferase from rhodococcus erythropolis MP50 is used as the acyl transferase.

4. Method according to claim 2 or 3, **characterised in that** the acyl transferase is used in the form of a raw extract from the microorganisms.

5. Method according to claim 2 or 3, **characterised in that** the acyl transferase is used in purified form.

6. Method according to claims 1 to 5, **characterised in that** the conversion is carried out in the presence of a buffer at a pH value of 6.5 to 7.5.

7. Method according to claims 1 to 6, **characterised in that** the residues R¹, R², and R³ are selected from the group comprising methyl, ethyl, n-propyl, i-propyl, n-butyl, i-butyl, t-butyl, n-pentyl, cyclohexyl, phenyl, t-butoxyphenyl, naphthyl, 3-benzoylphenyl, amino, hydroxy.

8. Method according to claim 7, **characterised in that** alkylaminopropionic acid, p-butoxyphenylacetic acid, 2-phenylpropionic acid, 2-methylbutanoic acid, 2-(1-naphthyl)propionic acid, 2-(6-methoxy-2-naphthyl) propionic acid (naproxen), 2-(3'-benzoylphenyl) propionic acid (ketoprofen) and esters or amides thereof are used as the compound of formula (I).

9. Method according to claims 1 to 8, **characterised in that** an amide is used as the compound of the general formula (I).

10. Method according to claims 1 to 9, **characterised in that** the compound of the general formula (1) is converted with 2 to 10 times the amount, preferably 5 times the amount, of hydroxylamine with an acyl transferase activity of 3000 to 5000 units per mol substrate, preferably 5000 units per mol substrate.

11. Method of producing optically active primary amines of the general formula: 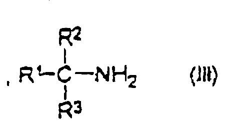 in which R¹, R² and R³ are as defined in claim 1, **characterised in that** the optically active hydroxamic acid of the general formula (I), obtained in the method according to claims 1 to 10, is O-acylated and the O-acylated hydroxamic acid is converted by heating or by treatment with bases in aprotic solvents to form the corresponding isocyanate and the isocyanate is caused to react with water with separation of CO₂ to form the amine of the general formula (III).

12. Method of producing optically active primary amines of the general formula: 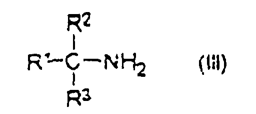 in which R¹, R² and R³ are defined as in claim 1, **characterised in that** the optically active hydroxamic acid of the general formula (I), obtained in the method according to claims 1 to 10, is converted before its separation from the non-converted enantiomers of the general formula (II) with a carbodiimide of the general formula: 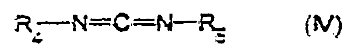 in which R₄ stands for benzyl, cyclohexyl or ethyl and R₅ for dimethyl aminopropyl or morpholinoethyl and then the optically active primary amine formed, of the general formula (III), is separated from the non-converted enantiomers of the general formula (II).

13. Method according to claim 12, **characterised in that** the reaction mixture containing the optically active hydroxamic acid (I) and the non-converted enantiomers (II) is acidulated before being converted with the carbodiimide to a pH value of 0 to 2, preferably to a pH value of 1, the reaction mixture is then heated to a temperature of between 40°C and 100°C, preferably 80°C, and the conversion with the carbodiimide is carried out thereafter at a pH value of 4 to 6, preferably a pH value of 5.0.

14. Method according to claim 12 or 13, **characterised in that** that 1-ethyl-3-(3'-dimethylaminopropyl) carbodiimide is used as the carbodiimide.

## Revendications

1. Procédé pour la préparation d'acides hydroxamiques optiquement actifs de formule générale 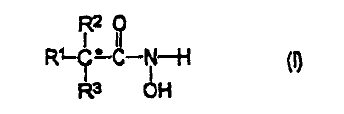 dans laquelle R¹, R² et R³ sont différents et représentent un radical hydrocarboné cyclique ou linéaire, aliphatique ou aromatique, substitué ou non substitué, qui peut éventuellement comporter des hétéroatomes,
**caractérisé en ce qu'**
on fait réagir un racémate d'amides chiraux, d'esters d'acides carboxyliques ou d'acides carboxyliques de formule générale 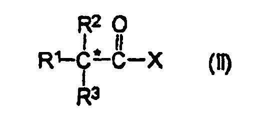 dans laquelle R¹, R² et R³ sont tels que définis plus haut, et X représente -NH₂, -OR ou -OH, R étant un radical organique quelconque,
avec de l'hydroxylamine NH₂OH, en présence d'une acyltransférase, et on sépare ensuite l'acide hydroxamique (I) optiquement actif formé, d'avec l'énantiomère de formule générale (II) n'ayant pas réagi.

2. Procédé selon la revendication 1,
**caractérisé en ce qu'**
on utilise comme acyltransférase une acyltransférase provenant des micro-organismes du groupe des *Mycobacteriaceae, Mycobacterium smegmatis, Pseudomonas aeruginosa, Arthrobacter, Aspergillus nidulans, Bradyrhizobium japonicum, Brevibacterium sp.* R312, *Methylophilus methylotrophus, Pseudomonas chlororaphis, Pseudomonas fluorescens, Rhodococcus rhodochrous* J1 et *Rhodococcus erythropolis* MP50.

3. Procédé selon la revendication 2,
**caractérisé en ce qu'**
on utilise comme acyltransférase l'acyltransférase de *Rhodococcus erythropolis* MP50.

4. Procédé selon la revendication 2 ou 3,
**caractérisé en ce qu'**
on utilise l'acyltransférase sous forme d'un extrait brut provenant des micro-organismes.

5. Procédé selon la revendication 2 ou 3,
**caractérisé en ce qu'**
on utilise l'acyltransférase sous forme purifiée.

6. Procédé selon les revendications 1 à 5,
**caractérisé en ce qu'**
on effectue la réaction en présence d'un tampon à un pH de 6,5 à 7,5.

7. Procédé selon les revendications 1 à 6,
**caractérisé en ce qu'**
on choisit les radicaux R¹, R² et R³ dans l'ensemble constitué par les groupes méthyle, éthyle, n-propyle, isopropyle, n-butyle, isobutyle, tert-butyle, n-pentyle, cyclohexyle, phényle, tert-butoxyphényle, naphtyle, 3-benzoylphényle, amino, hydroxy.

8. Procédé selon la revendication 7
**caractérisé en ce qu'**
on utilise comme composé de formule (I) un acide alkylaminopropionique, l'acide p-butoxyphénylacétique, l'acide 2-phénylpropionique, l'acide 2-méthylbutanoique, l'acide 2-(1-naphtyl)propionique, l'acide 2-(6-méthoxy-2-naphtyl)propionique (naproxène), l'acide 2-(3'-benzoylphényl)propionique (kétoprofène) et leurs esters ou amides.

9. Procédé selon les revendications 1 à 8,
**caractérisé en ce qu'**
on utilise comme composé de formule générale (I) un amide.

10. Procédé selon les revendications 1 à 9,
**caractérisé en ce qu'**
on fait réagir le composé de formule générale (I) avec 2 à 10 fois sa quantité, de préférence 5 fois sa quantité d'hydroxylamine, à une activité acyltransférase de 3 000 à 5 000 unités par mole de substrat, de préférence de 5 000 unités par mole de substrat.

11. Procédé pour la préparation d'amines primaires optiquement actives de formule générale : 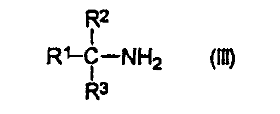 dans laquelle R¹, R² et R³ sont tels que définis dans la revendication 1,
**caractérisé en ce qu'**
on soumet à une O-acylation l'acide hydroxamique optiquement actif de formule générale (I), obtenu dans le procédé selon les revendications 1 à 10, et l'acide hydroxamique O-acylé est converti en l'isocyanate correspondant, par chauffage ou par traitement par des bases dans des solvants aprotiques, et on fait réagir l'isocyanate avec de l'eau, avec élimination de CO₂, pour aboutir à l'amine de formule générale (III).

12. Procédé pour la préparation d'amines primaires optiquement actives de formule générale : 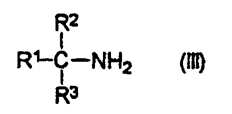 dans laquelle R¹, R² et R³ sont tels que définis dans la revendication 1,
**caractérisé en ce qu'**
on fait réagir l'acide hydroxamique optiquement actif de formule générale (I), obtenu dans le procédé selon les revendications 1 à 10, avant sa séparation de l'énantiomère de formule générale (II) n'ayant pas réagi, avec un carbodiimide de formule générale
R₄-N=C=N-R₅ (IV)
dans laquelle R₄ représente le groupe benzyle, cyclohexyle ou éthyle, et R₅ représente le groupe diméthylaminopropyle ou morpholinoéthyle, et on sépare ensuite l'amine primaire optiquement active formée, de formule générale (III), d'avec l'énantiomère de formule générale (II) n'ayant pas réagi.

13. Procédé selon la revendication 12,
**caractérisé en ce qu'**
on acidifie à un pH de 0 à 2, de préférence à pH=1, le mélange réactionnel contenant l'acide hydroxamique optiquement actif (I) et l'énantiomère (II) n'ayant pas réagi, avant sa réaction avec le carbodiimide, on chauffe ensuite le mélange réactionnel à une température de 40°C à 100°C, de préférence de 80°C, et on effectue ensuite la réaction avec le carbodiimide à un pH de 4 à 6, de préférence à un pH de 5,0.

14. Procédé selon la revendication 12 ou 13,
**caractérisé en ce qu'**
on utilise comme carbodiimide le 1-éthyl-3-(3'-diméthylaminopropyl)-carbodiimide.
